(19) **Europäisches Patentamt / European Patent Office / Office européen des brevets**

(11) **EP 1 958 596 B1**

(12) **FASCICULE DE BREVET EUROPEEN**

(45) Date de publication et mention de la délivrance du brevet:
**09.07.2014 Bulletin 2014/28**

(51) Int Cl.:
**A61F 2/36** (2006.01)

(21) Numéro de dépôt: **08151130.5**

(22) Date de dépôt: **06.02.2008**

(54) **Tige fémorale pour prothèse de hanche**

Oberschenkelhals für Hüftprothese

Femoral rod for a hip prosthesis

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **14.02.2007 FR 0753252**

(43) Date de publication de la demande:
**20.08.2008 Bulletin 2008/34**

(73) Titulaires:
- **Biomet Merck France**
  **26903 Valence Cedex (FR)**
- **Basseres, Bruno**
  **30000 Nimes (FR)**
- **Benareau, Ignaki**
  **83400 Hyeres (FR)**
- **Besson, Christophe**
  **38190 Bernin (FR)**
- **Cambuzat, Arnaud**
  **74200 Armoy (FR)**
- **Cladiere, Frank**
  **07500 Guilherand-Granges (FR)**
- **Fornasieri, Christophe**
  **38240 Meylan (FR)**
- **Guillaumie, Benoît**
  **6900 Marche en Famenne (BE)**
- **Lamy, Henri**
  **39100 Dole (FR)**
- **Lantuejoul, Jean-Pierre**
  **38300 Saint-Ismier (FR)**
- **Lenfant, Jacques**
  **75017 Paris (FR)**
- **Leroy, Jean-Michel**
  **74200 Anthy Sur Leman (FR)**
- **Montbarbon, Eric**
  **73000 Barberaz (FR)**

- **Rizk, Serge**
  **71530 Lessard Le National (FR)**
- **Van Nieuwenhuyse, Nicolas M.**
  **74380 Lucinges (FR)**
- **Vinciguerra, Bruno**
  **64600 Anglet (FR)**

(72) Inventeurs:
- **Basseres, Bruno**
  **30000, NIMES (FR)**
- **Benareau, Ignaki**
  **83400, HYERES (FR)**
- **Besson, Christophe**
  **38190, BERNIN (FR)**
- **Cambuzat, Arnaud**
  **74200, ARMOY (FR)**
- **Cladiere, Frank**
  **07500, GUILHERAND-GRANGES (FR)**
- **Fornasieri, Christophe**
  **38240, MEYLAN (FR)**
- **Gaullaumie, Bênoit**
  **6900, MARCHE EN FAMENNE (BE)**
- **Lamy, Henri**
  **39100, DOLE (FR)**
- **Lantuejoul, Jean-Pierre**
  **38300, SAINT-ISMIER (FR)**
- **Lenfant, Jacques**
  **75017, PARIS (FR)**
- **Leroy,Jean-Michel**
  **74200, ANTHY SUR LEMAN (FR)**
- **Montbarbon, Eric**
  **73000, BARBERAZ (FR)**
- **Rizk, Serge**
  **71530, LESSARD LE NATIONAL (FR)**
- **Van Nieuwenhuyse, Nicolas**
  **74380, LUCINGES (FR)**

- **Vinciguerra, Bruno**
  **64600, ANGLET (FR)**
- **Roques, Elise**
  **07500, GUILHERAND-GRANGES (FR)**
- **Vernizeau, Michel**
  **26120, UPIE (FR)**

(74) Mandataire: **Blanchard, Eugène Gilles**
     **Cabinet Beau de Loménie**
     **51, avenue Jean Jaurès**
     **B.P. 7073**
     **69301 Lyon Cedex 07 (FR)**

(56) Documents cités:
     **FR-A1- 2 784 576     FR-A1- 2 844 994**
     **FR-A1- 2 849 770**

**Description**

La présente invention concerne le domaine des implants orthopédiques et, plus particulièrement, des prothèses mises en oeuvre pour réhabiliter les articulations du corps humain.

L'objet de l'invention est plus particulièrement dirigé vers les prothèses de hanche qui sont mises en place après résection du col du fémur, dans les interventions destinées à réhabiliter l'articulation coxo-fémorale.

Dans le domaine technique ci-dessus, il est maintenant habituel d'implanter des prothèses de hanche qui ont fait l'objet de nombreuses propositions techniques, en vue d'assurer une réhabilitation dans les meilleures conditions et pour des durées les plus longues possibles.

Il serait vain de vouloir établir un rappel exhaustif de toutes les propositions connues à ce jour, en raison de leur grand nombre. Il peut simplement être rappelé que les prothèses de hanche peuvent être, généralement, classées en deux catégories selon qu'elles sont implantées avec ou sans présence d'un liant intermédiaire, tel qu'un ciment.

L'objectif d'une prothèse est donc d'assurer une parfaite réhabilitation de l'articulation de la hanche tout en procurant un confort satisfaisant au patient, c'est-à-dire en réduisant autant que faire se peut les douleurs fonctionnelles au niveau des zones d'implantation et en réduisant les risques de rupture de l'os recevant la prothèse.

Or, il est apparu que les prothèses selon l'art antérieur n'étaient pas toujours en mesure d'atteindre ces objectifs et il a été cherché une optimisation de la prothèse de hanche permettant notamment de réduire mieux encore les douleurs fonctionnelles telles que ligamentaires et osseuses intervenant bien après la consolidation de l'implantation.

Afin d'atteindre ces objectifs, l'invention concerne une prothèse de hanche comprenant une tige liée par un col dont l'extrémité est destinée à recevoir une tête d'articulation, la tige comportant, à partir du col, une partie massive, dite métaphysaire, prolongée par une queue, et possédant un axe général A-A' faisant un angle $\alpha$ avec l'axe B-B' du col, caractérisée en ce que l'angle $\alpha$ possède, en projection dans un plan frontal **F,** une valeur, exprimée en degrés,qui vérifie la relation suivante :

$$\alpha = 0{,}1376\ \mathbf{L} + 108{,}3648 \pm \mathbf{e}$$

où :

■ **L** est la longueur de la tige mesurée parallèlement à l'axe **A-A'** entre un épaulement perpendiculaire à l'axe **A-A'** et l'extrémité inférieure de la tige,
■ **e** est une tolérance valant 0,3°, de préférence 0,16° et, de manière plus particulièrement préférée, 0,09°;
■ le col possède une longueur **lc,** mesurée entre son extrémité opposée à la tige et la base de raccordement du col à la tige, qui, exprimée en millimètres, vérifie la relation suivante :

$$\mathbf{lc} = 0{,}248\ \mathbf{L} - 1{,}5 \pm \mathbf{e'}$$

où :

■ **e'** est une tolérance valant 0,6 mm, de préférence 0,3 mm, et, de manière plus particulièrement préférée, 0,25 mm.

Les inventeurs ont en effet eu le mérite de montrer qu'afin d'obtenir une meilleure adéquation entre la prothèse et la physionomie du patient, et donc un plus grand confort, il convient d'adopter un angle entre le col et l'axe de la tige différent en fonction de la taille de la tige. Les inventeurs ont par ailleurs mis en évidence que la proportionnalité entre, d'une part, l'angle du col et sa longueur et, d'autre part, la taille ou longueur de la tige permet une réduction des douleurs fonctionnelles engendrées par la prothèse.

De manière préférée, la valeur de l'angle $\alpha$ sera comprise entre 124° et 132° et, de préférence, comprise entre 125,3° et 130,6° de sorte qu'une prothèse selon l'invention possédera à longueur de tige équivalente un angle $\alpha$ entre l'axe de la tige et l'axe du col plus fermé que celui d'une tige selon l'art antérieur. Or, les inventeurs ont démontrés qu'une trop grande ouverture de col pouvait également être à l'origine de douleur fonctionnelles.

Selon une autre caractéristique, la prothèse de hanche selon l'invention comprend un bord, dit externe, qui prend naissance à un épaulement, sensiblement perpendiculaire à l'axe A-A', du col et se prolongeant jusqu'à l'extrémité inférieure de la queue et qui est de forme générale convexe.

Dans une forme préférée mais non exclusive de réalisation de la prothèse de hanche selon l'invention, le bord externe comprend alors une partie proximale et une partie distale qui forment en vue de profil un angle obtus $\beta$ qui

possède une valeur comprise entre 161° et 180° et, de préférence, comprise entre 162,1° et 178,9°.

Afin d'optimiser l'adéquation entre la forme de la tige et la forme du fémur d'implantation, la valeur de l'angle β, exprimée en degrés, vérifie de préférence la relation suivante :

$$\beta = 9{,}1584\ \mathbf{L} + 166{,}8509 \pm \mathbf{e''}$$

où :

■ **L** est la longueur de la tige mesurée parallèlement à l'axe **A-A'** entre un épaulement perpendiculaire à l'axe **A-A'** et l'extrémité inférieure de la tige,
■ **e''** est une tolérance valant 6,6°, de préférence 5,5° et, de manière plus particulièrement préférée, 3,9°.

Selon une caractéristique de l'invention, la largeur $\ell$, de la tige de la prothèse de hanche, mesurée perpendiculairement à l'axe A-A' à partir de la jonction entre les parties proximale et distale du bord externe et jusqu'au bord interne opposé, présente une valeur comprise entre 16 mm et 31 mm et de préférence comprise entre 17 mm et 29,4 mm.

Toujours en vue d'assurer une meilleure adéquation de la prothèse au fémur du patient en fonction notamment de la taille de prothèse retenue, la largeur $\ell$ sera de préférence proportionnelle à la longueur de la tige et, de manière préférée, la largeur $\ell$ de la tige, exprimée en millimètres, vérifie la relation suivante :

$$\ell = 0{,}323\ L - 22{,}852 \pm e'''$$

où :

■ L est la longueur de la tige mesurée parallèlement à l'axe A-A' entre un épaulement du col perpendiculaire à l'axe A-A' et l'extrémité inférieure de la tige,
■ e''' est une tolérance valant 0,6 mm, de préférence 0,4 mm, et, de manière plus particulièrement préférée, 0,25 mm.

Selon une autre caractéristique de l'invention visant à permettre notamment une bonne immobilisation de la prothèse sans avoir à prendre appui sur l'os dans lequel elle est implantée, le col selon l'invention comprend deux méplats, respectivement antérieur et postérieur, qui s'étendent vers le cône à partir d'une base plane de raccordement entre le col et la tige.

Selon encore une autre caractéristique de l'invention, la longueur L de la tige est comprise entre 125 mm et 162 mm.

Selon encore une autre caractéristique de l'invention visant à faciliter l'adaptation de la tête d'articulation sur le col, l'extrémité du col forme un cône d'adaptation de la tête d'articulation tel que par exemple un cône morse.

Afin de permettre à un chirurgien de disposer d'une prothèse la plus adaptée possible à la morphologie de chaque patient, l'invention concerne également un ensemble de prothèses de hanche, de tailles différentes comprenant au moins deux prothèses de hanche de longueur de tige distinctes, la longueur de chacune des prothèses de la gamme appartenant à une catégorie différente et choisie parmi les catégories suivantes :

■ T1 : longueur de tige L comprise entre 125 mm et 126 mm,
■ T2 : longueur de tige L comprise entre 128 mm et 129 mm,
■ T3 : longueur de tige L comprise entre 131 mm et 132mm,
■ T4 : longueur de tige L comprise entre 136 mm et 137 mm,
■ T5 : longueur de tige L comprise entre 141 mm et 142 mm,
■ T6 : longueur de tige L comprise entre 147 mm et 148 mm,
■ T7 : longueur de tige L comprise entre 152 mm et 153 mm,
■ T8 : longueur de tige L comprise entre 156 mm et 158 mm,
■ T9 : longueur de tige L comprise entre 161 mm et 162 mm,
la longueur L de la tige étant mesurée parallèlement à l'axe A-A' entre un épaulement du col perpendiculaire à l'axe A-A' et l'extrémité inférieure de la tige.

Bien entendu, les différentes caractéristiques de l'invention évoquées ci-dessus peuvent être mises en oeuvre les unes avec les autres selon différentes combinaisons lorsqu'elles ne sont pas exclusives les unes des autres.

Par ailleurs, diverses autres caractéristiques de l'invention ressortent de la description faite ci-dessous en référence aux dessins annexés qui montrent, à titre d'exemples non limitatifs, des formes de réalisation de l'objet de l'invention.

La **fig. 1** est une élévation d'une prothèse gauche selon l'invention montrant la face antérieure de la prothèse, c'est-à-dire celle orientée vers l'avant du patient une fois la prothèse implantée.

La **fig. 2** est une vue de gauche, selon la ligne II-II de la fig. 1, de la prothèse selon l'invention.

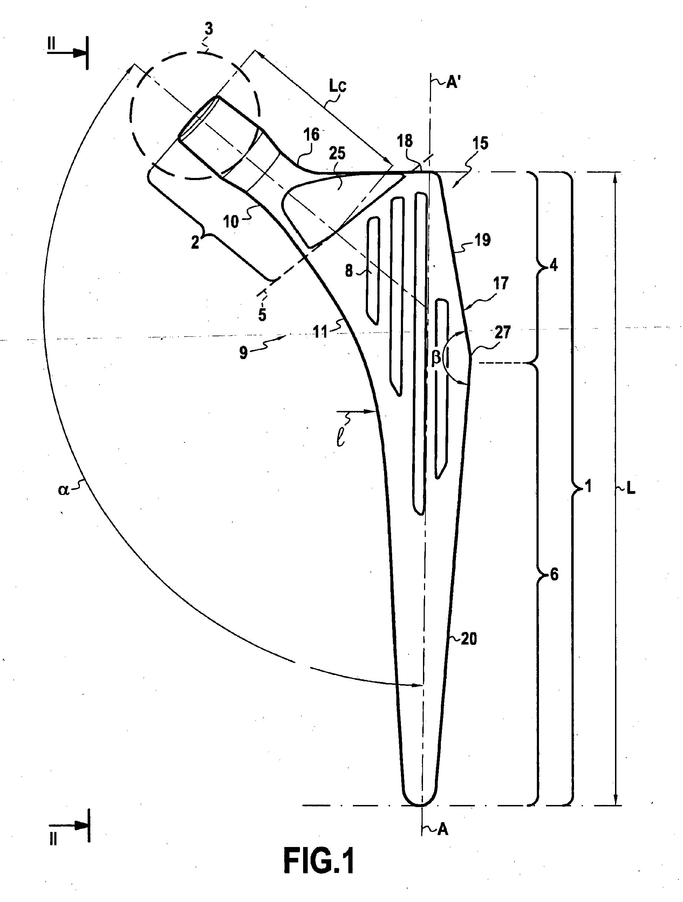

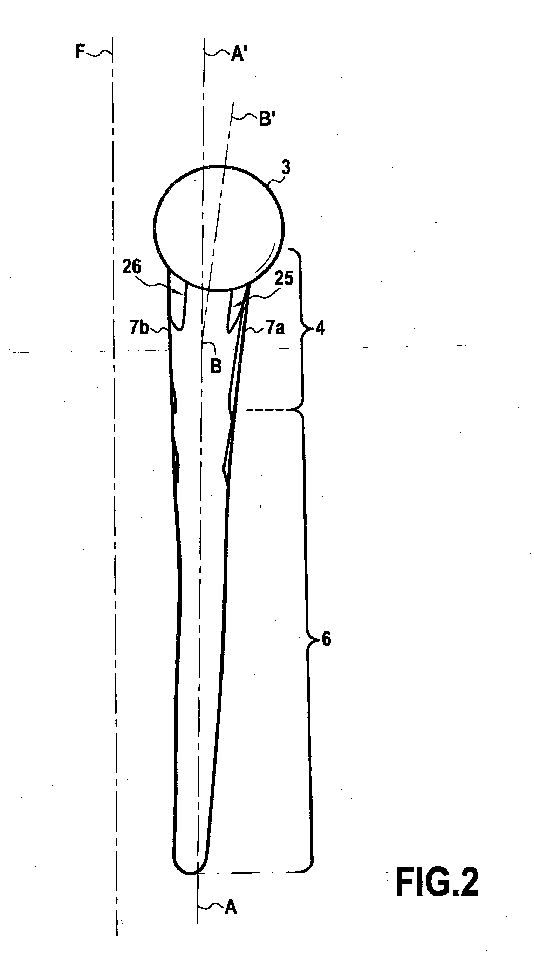

Comme le montrent les **fig. 1** et **2**, une prothèse de hanche selon l'invention comprend une tige **1** qui est liée à un col **2** destiné à recevoir une tête prothétique **3**.

Au sens de l'invention, il peut être considéré indifférent que la tête **3** fasse partie intégrante de la prothèse ou soit constituée par une tête sphérique rapportée à l'extrémité du col, comme cela est illustré par le dessin. A cet effet, et selon l'exemple illustré, l'extrémité du col **2** est réalisée sous la forme d'un cône morse.

La prothèse est réalisée de manière que la tige **1** comporte une partie épaisse ou massive **4**, dite métaphysaire, s'étendant à partir d'une base **5** de raccordement avec le col **2** et se prolongeant par une queue **6**.

La partie massive métaphysaire **4** est définie par deux faces, antérieure **7a** et postérieure **7b**, qui convergent légèrement l'une vers l'autre en direction de l'extrémité inférieure de la tige. Bien que la tige **1** puisse être conformée pour présenter une courbure appropriée au fémur dans lequel elle doit être implantée, il est considéré, au sens de l'invention, que la tige s'étend le long d'un axe longitudinal rectiligne **A-A'** tandis que le col s'étend selon un axe **B-B'**. Conformément à une caractéristique essentielle de l'invention, l'axe **A-A'** de la tige forme avec l'axe **B-B'** du col un angle $\alpha$ qui possède, en projection dans un plan frontal **F**, illustré **fig. 2**, une valeur comprise entre 124° et 132° et, de préférence, comprise entre 125,3° et 130,6°.

Les faces planes **7a** et **7b** présentent, chacune, des stries, rainures ou analogues **8**, établies longitudinalement et parallèlement entre elles, de façon à favoriser l'ancrage ultérieur de la masse osseuse de reconstitution. Certaines des stries ou rainures **8** se prolongent au niveau de leurs extrémités distales sur les faces antérieures et postérieures correspondantes de la queue **6**.

A cet égard, la partie métaphysaire peut, avantageusement, faire l'objet d'un apport d'un revêtement d'hydroxyapatite de calcium. Les stries **8** sont du type non débouchant en extrémité transversale sur le col, afin d'éviter toute migration de particules vers le haut. Par contre, en extrémité distale, les stries **8** sont "fuyantes", c'est-à-dire qu'elles tendent à se raccorder à la surface des faces **7a**, **7b**, de manière à faciliter une rétraction future en cas de besoin.

La tige est délimitée, transversalement aux faces **7a** et **7b**, par un bord interne **9**, reliant l'extrémité réelle tronconique du col **2** à l'extrémité inférieure de la tige **1**. Le bord interne comprend alors une partie proximale **10** de forme concave et formée par le col **2**, ainsi qu'une partie distale **11** de forme également concave et formée par la tige **1**.

La prothèse est également délimitée à l'opposé du bord interne par un bord **15**, dit externe, comprenant une partie concave **16** formée par le col et une partie convexe **17** formée par la tige **1**. La partie convexe **17** du bord externe de la tige **1** prend naissance à partir d'un épaulement **18** perpendiculaire à l'axe **A-A'** se prolongeant au niveau du col **2**. Le bord externe **17** comprend en outre une partie proximale **19** prolongée jusqu'à l'extrémité inférieure de la tige par une partie distale **20**. La partie proximale **19** et la partie distale **20** forment alors, en vue de profil telle qu'illustrée à la **fig. 1** ou en projection dans le plan frontal **F**, un angle obtus $\beta$ qui possède, une valeur comprise entre 161° et 180° et, de préférence, comprise entre 162,1° et 178,9°.

Afin de faciliter notamment la mise en place et le retrait de la tête **3**, le col **2** présente à sa base deux méplats antérieur **25** et postérieur **26** qui s'étendent chacun vers l'extrémité du col **2** à partir d'une base plane de raccordement entre le col **2** et la tige **1**.

Selon notre caractéristique de l'invention, le col **2** possède une longueur **lc**, mesurée entre son extrémité opposée à la tige **1** et la base de raccordement du col **2** à la tige, qui présente une valeur comprise entre 31 mm et 43 mm et, de préférence, comprise entre 32 mm et 42 mm.

Par ailleurs, la tige **1** aura, de préférence, une longueur **L** mesurée parallèlement à l'axe **A-A'** entre un épaulement du col perpendiculaire à l'axe **A-A'** et l'extrémité inférieure de la tige, comprise entre 125 mm et 162 mm.

De plus, la tige **1** présentera, de préférence, une largeur $\ell$, mesurée perpendiculairement à l'axe **A-A'** à partir de la jonction **27** entre les parties proximale **19** et distale **20** du bord externe et jusqu'au bord interne opposé, d'une valeur comprise entre 16 mm et 31 mm et, de préférence, comprise entre 17 mm et 29,4 mm.

Selon l'invention, il existe une proportionnalité entre, d'une part, la longueur **L** de la tige **1** et, d'autre part, la longueur **lc** du col, l'angle $\alpha$, ainsi que de préférence la largeur $\ell$ de la tige et l'angle $\beta$, de sorte qu'il est possible, pour chaque taille de tige, d'obtenir une prothèse la plus proche possible de la morphologie du patient.

Les relations de proportionnalité que les inventeurs ont eu le mérite de mettre en évidence sont alors les suivantes :

$$\alpha = 0{,}1376\, \mathbf{L} + 108{,}3648 \pm \mathbf{e}$$

OÙ :

■ **L** est la longueur de la tige mesurée parallèlement à l'axe **A-A'** entre un épaulement du col perpendiculaire à l'axe **A-A'** et l'extrémité inférieure de la tige,
■ **e** est une tolérance valant 0,3°, de préférence 0,16° et, de manière plus particulièrement préférée 0,09°.

$$lc = 0,248\ \mathbf{L} - 1,5 \pm \mathbf{e'}$$

où :

■ **e** est une tolérance valant 0,6 mm, de préférence 0,3 mm, et, de manière plus particulièrement, préférée 0,25 mm,

$$\boldsymbol{\beta} = 9,1584\ \mathbf{L} + 166,8509 \pm \mathbf{e''}$$

où :

■ **e"** est une tolérance valant 6,6°, de préférence 5,5° et, de manière plus particulièrement préférée, 3,9°.

$$\ell = 0,323\ \mathbf{L} - 22,852 \pm \mathbf{e'''}$$

où :

■ **e"'** est une tolérance valant 0,6 mm, de préférence 0,4 mm, et, de manière plus particulièrement préférée, 0,25 mm.

Ces relations de proportionnalité peuvent alors être mises à profit pour concevoir une gamme de deux tiges selon l'invention de différentes tailles inscrivant par exemple dans les gammes suivantes :

■ T1 : longueur de tige L comprise entre 125 mm et 126 mm,
■ T2 : longueur de tige L comprise entre 128 mm et 129 mm,
■ T3 : longueur de tige L comprise entre 131 mm et 132mm,
■ T4 : longueur de tige L comprise entre 136 mm et 137 mm,
■ T5 : longueur de tige L comprise entre 141 mm et 142 mm,
■ T6 : longueur de tige L comprise entre 147 mm et 148 mm,
■ T7 : longueur de tige L comprise entre 152 mm et 153 mm,
■ T8 : longueur de tige L comprise entre 156 mm et 158 mm,
■ T9 : longueur de tige L comprise entre 161 mm et 162 mm,

Bien entendu, l'invention n'est pas limitée à l'exemple décrit et représenté car diverses modifications peuvent y être apportées sans sortir de son cadre.

**Revendications**

1. Prothèse de hanche comprenant une tige (**1**) liée par un col (**2**) dont l'extrémité est destinée à recevoir une tête d'articulation (**3**), la tige (**1**) comportant, à partir du col (**2**), une partie massive (**4**), dite métaphysaire, prolongée par une queue (**6**), et possédant un axe général (**A-A'**) faisant un angle ($\alpha$) avec l'axe (**B-B'**) du col, **caractérisée en ce que** :

■ l'angle $\alpha$ possède, en projection dans un plan frontal **F**, une valeur, exprimée en °, qui vérifie la relation suivante :

$$\alpha = 0,1376\ \mathbf{L} + 108,3648 \pm \mathbf{e}$$

où :

■ **L** est la longueur de la tige (**1**) mesurée parallèlement à l'axe **A-A'** entre un épaulement (**18**) perpendiculaire à l'axe **A-A'** et l'extrémité inférieure de la tige (**1**),
■ **e** est une tolérance valant 0,3°, de préférence 0,16° et, de manière plus particulièrement préférée, 0,09°;

■ lé col (**2**) possède une longueur **lc**, mesurée entre son extrémité opposée à la tige (**1**) et la base de raccordement du col à la tige, qui, exprimée en mm, vérifie la relation suivante :

$$\mathbf{lc} = 0,248\ \mathbf{L} - 1,5 \pm \mathbf{e'}$$

où:

■ **e**' est une tolérance valant 0,6 mm, de préférence 0,3 mm, et, de manière plus particulièrement préférée, 0,25 mm..

2. Prothèse de hanche selon la revendication 1 **caractérisée en ce que** la valeur de l'angle $\alpha$ est comprise entre 124° et 132° et, de préférence, comprise entre 125,3° et 130,6°.

3. Prothèse de hanche selon la revendication 1 ou 2, **caractérisée en ce que** la longueur **lc** du col (**2**) présente une valeur comprise entre 31 mm et 43 mm et, de préférence, comprise entre 32 mm et 42 mm.

4. Prothèse de hanche selon l'une des revendications 1 à 3 **caractérisée en ce qu'**elle comprend un bord (**17**), dit externe, prenant naissance à un épaulement (**18**), sensiblement perpendiculaire à l'axe **A-A'** du col et se prolongeant jusqu'à l'extrémité inférieure de la queue (**6**) et qui est de forme générale convexe.

5. Prothèse de hanche selon la revendication 4, **caractérisée en ce que** le bord externe (**17**) comprend une partie proximale (**19**) et une partie distale (**20**) qui forme en vue de profil un angle obtus $\beta$ qui possède, en projection dans un plan frontal **F**, une valeur comprise entre 161° et 180° et, de préférence, comprise entre 162,1° et 178,9°.

6. Prothèse de hanche selon la revendication 5, **caractérisée en ce que** la valeur de l'angle $\beta$, exprimée en °, qui vérifie la relation suivante :

$$\beta = 9,1584\ \mathbf{L} + 166,8509 \pm \mathbf{e''}$$

où :

■ **L** est la longueur de la tige (**1**) mesurée parallèlement à l'axe **A-A'** entre un épaulement (**18**) perpendiculaire à l'axe **A-A'** et l'extrémité inférieure de la tige (**1**),
■ **e"** est une tolérance valant 6,6°, de préférence 5,5° et, de manière plus particulièrement préférée, 3,9°.

7. Prothèse de hanche selon la revendication 5 ou 6, **caractérisée en ce que** la largeur $\ell$ de la tige (**1**), mesurée perpendiculairement à l'axe **A-A'** à partir de la jonction (**27**) entre les parties proximale (**19**) et distale (**20**) du bord externe (**17**) et jusqu'au bord interne opposé (**11**), présente une valeur comprise entre 16 mm et 31 mm et, de préférence, comprise entre 17 mm et 29,4 mm.

8. Prothèse de hanche selon la revendication 7, **caractérisée en ce que** la largeur $\ell$ de la tige (**1**), exprimée en mm, vérifie la relation suivante :

$$\ell = 0,323\ \mathbf{L} - 22,852 \pm \mathbf{e'''}$$

où :

■ **L** est la longueur de la tige (**1**) mesurée parallèlement à l'axe **A-A'** entre un épaulement (**18**) perpendiculaire à l'axe **A-A'** et l'extrémité inférieure de la tige,
■ **e'''** est une tolérance valant 0,6 mm, de préférence 0,4 mm, et, de manière plus particulièrement préférée, **0,25** mm.

9. Prothèse de hanche selon l'une des revendications 1 à 8 **caractérisée en ce que** le col comprend deux méplats (**25,26**), respectivement antérieur et postérieur, qui s'étendent à partir d'une base plane de raccordement entre le col (**2**) et la tige (**1**) vers une extrémité opposée du col (**2**).

10. Prothèse de hanche selon l'une des revendications 1 à 9, **caractérisée en ce que** la longueur **L** de la tige (**1**) est comprise entre 125 mm et 162 mm.

11. Prothèse de hanche selon l'une des revendications 1 à 10 **caractérisée en ce que** l'extrémité du col (**2**) forme un cône d'adaptation de la tête d'articulation.

12. Ensemble de prothèses de hanche selon l'une des revendications 1 à 11, de tailles différentes comprenant au moins deux prothèses de hanche de longueur de tige distinctes, la longueur **L** de la tige de chacune des prothèses de la gamme appartenant à une catégorie différente et choisie parmi les catégories suivantes :

   ■ T1 : longueur de tige L comprise entre 125 mm et 126 mm,
   ■ T2 : longueur de tige L comprise entre 128 mm et 129 mm,
   ■ T3 : longueur de tige L comprise entre 131 mm et 132mm,
   ■ T4 : longueur de tige L comprise entre 136 mm et 137 mm,
   ■ T5 : longueur de tige L comprise entre 141 mm et 142 mm,
   ■ T6 : longueur de tige L comprise entre 147 mm et 148 mm,
   ■ T7 : longueur de tige L comprise entre 152 mm et 153 mm,
   ■ T8 : longueur de tige L comprise entre 156 mm et 158 mm,
   ■ T9 : longueur de tige L comprise entre 161 mm et 162 mm,

la longueur **L** de la tige étant mesurée parallèlement à l'axe **A-A'** entre un épaulement du col perpendiculaire à l'axe **A-A'** et l'extrémité inférieure de la tige.

## Patentansprüche

1. Hüftprothese, umfassend einen Schaft (1), der über einen Hals (2) verbunden ist, dessen Ende dazu bestimmt ist, einen Gelenkkopf (3) aufzunehmen, wobei der Schaft (1) ausgehend von dem Hals (2) einen massiven, sogenannten metaphysären Teil (4) umfasst, der durch ein Endstück (6) fortgesetzt ist, und eine Hauptachse (A-A') besitzt, die mit der Achse (B-B') des Halses einen Winkel (α) bildet, **dadurch gekennzeichnet, dass**:

   - der Winkel α in einer Projektion in einer Frontalebene F einen Wert, ausgedrückt in °, besitzt, der die folgende Beziehung erfüllt:

$$\alpha = 0,1376\ \mathrm{L} + 108,3648 \pm e$$

   worin:

      - L die Länge des Schaftes (1), gemessen parallel zur Achse A-A' zwischen einer zu der Achse (A-A') senkrechten Schulter (18) und dem unteren Ende des Schaftes (1), ist,
      - e eine Toleranz von 0,3°, vorzugsweise 0,16° und besonders bevorzugt 0,09° ist,

- der Hals (2) eine Länge Ic, gemessen zwischen seinem von dem Schaft (1) abgewandten Ende und der Basis zum Anschließen des Halses an den Schaft, besitzt, die, in mm ausgedrückt, die folgende Beziehung erfüllt:

$$Ic = 0{,}248\ L - 1{,}5 \pm e'$$

worin:

- e' eine Toleranz von 0,6 mm, vorzugsweise 0,3 mm und besonders bevorzugt 0,25 mm ist.

2. Hüftprothese nach Anspruch 1, **dadurch gekennzeichnet, dass** der Wert des Winkels $\alpha$ im Bereich zwischen 124° und 132° und vorzugsweise zwischen 125,3° und 130,6° liegt.

3. Hüftprothese nach Anspruch 1 oder 2, **dadurch gekennzeichnet, dass** die Länge Ic des Halses (2) einen Wert im Bereich zwischen 31 mm und 43 mm und vorzugsweise zwischen 32 mm und 42 mm aufweist.

4. Hüftprothese nach einem der Ansprüche 1 bis 3, **dadurch gekennzeichnet, dass** sie einen sogenannten äußeren Rand (17) umfasst, der an einer zu der Achse A-A' im Wesentlichen senkrechten Schulter (18) des Halses beginnt und sich bis zu dem unteren Ende des Endstücks (6) fortsetzt und der eine konvexe Gesamtform aufweist.

5. Hüftprothese nach Anspruch 4, **dadurch gekennzeichnet, dass** der äußere Rand (17) einen proximalen Teil (19) und einen distalen Teil (20) umfasst, der in Profilansicht einen stumpfen Winkel $\beta$ bildet, welcher in einer Projektion in einer Frontalebene F einen Wert im Bereich zwischen 161° und 180° und vorzugsweise zwischen 162,1° und 178,9° besitzt.

6. Hüftprothese nach Anspruch 5, **dadurch gekennzeichnet, dass** der Wert des Winkels $\beta$, ausgedrückt in °, die folgende Beziehung erfüllt:

$$\beta = 9{,}1584\ L + 166{,}8509 \pm e''$$

worin:

- L die Länge des Schaftes (1), gemessen parallel zur Achse A-A' zwischen einer zu der Achse (A-A') senkrechten Schulter (18) und dem unteren Ende des Schaftes (1), ist,
- e" eine Toleranz von 6,6°, vorzugsweise 5,5° und besonders bevorzugt 3,9° ist.

7. Hüftprothese nach Anspruch 5 oder 6, **dadurch gekennzeichnet, dass** die Breite $\ell$ des Schaftes (1), gemessen senkrecht zur Achse A-A' ab der Verbindung (27) zwischen dem proximalen (19) und dem distalen (20) Teil des äußeren Randes (17) und bis zum gegenüberliegenden inneren Rand (11), einen Wert im Bereich zwischen 16 mm und 31 mm und vorzugsweise zwischen 17 mm und 29,4 mm aufweist.

8. Hüftprothese nach Anspruch 7, **dadurch gekennzeichnet, dass** die Breite $\ell$ des Schaftes (1), ausgedrückt in mm, die folgende Beziehung erfüllt:

mm ausgedrückt die folgende Beziehung erfüllt:

$$\ell = 0{,}323\ L - 22{,}852 \pm e'''$$

worin:

- L die Länge des Schaftes (1), gemessen parallel zur Achse A-A' zwischen einer zu der Achse (A-A') senkrechten Schulter (18) und dem unteren Ende des Schaftes, ist,
- e''' eine Toleranz von 0,6 mm, vorzugsweise 0,4 mm und besonders bevorzugt 0,25 mm ist.

9. Hüftprothese nach einem der Ansprüche 1 bis 8, **dadurch gekennzeichnet, dass** der Hals zwei Abflachungen (25, 26), eine vordere bzw. eine hintere, umfasst, die sich ausgehend von einer ebenen Anschlussbasis zwischen dem Hals (2) und dem Schaft (1) in Richtung eines gegenüberliegenden Endes des Halses (2) erstrecken.

10. Hüftprothese nach einem der Ansprüche 1 bis 9, **dadurch gekennzeichnet, dass** die Länge L des Schaftes (1) zwischen 125 mm und 162 mm beträgt.

11. Hüftprothese nach einem der Ansprüche 1 bis 10, **dadurch gekennzeichnet, dass** das Ende des Halses (2) einen Konus zur Anpassung des Gelenkkopfes bildet.

12. Satz von Hüftprothesen nach einem der Ansprüche 1 bis 11, mit unterschiedlichen Größen, wenigstens umfassend zwei Hüftprothesen mit unterschiedlichen Schaftlängen, wobei die Länge L des Schaftes einer jeden der Prothesen der Palette zu einer anderen und aus den folgenden Kategorien ausgewählten Kategorie gehört:

   - T1: Schaftlänge L zwischen 125 mm und 126 mm,
   - T2: Schaftlänge L zwischen 128 mm und 129 mm,
   - T3: Schaftlänge L zwischen 131 mm und 132 mm,
   - T4: Schaftlänge L zwischen 136 mm und 137 mm,
   - T5: Schaftlänge L zwischen 141 mm und 142 mm,
   - T6: Schaftlänge L zwischen 147 mm und 148 mm,
   - T7: Schaftlänge L zwischen 152 mm und 153 mm,
   - T8: Schaftlänge L zwischen 156 mm und 158 mm,
   - T9: Schaftlänge L zwischen 161 mm und 162 mm,

   wobei die Länge L des Schaftes parallel zur Achse A-A' zwischen einer zu der Achse A-A' senkrechten Schulter des Halses und dem unteren Ende des Schaftes gemessen wird.

**Claims**

1. A hip prosthesis comprising a stem (1) connected by a neck (2) having an end that is to receive a head (3) for the joint, the stem (1) comprising, starting from the neck (2), a "metaphyseal" solid portion (4) extended by a tail (6) and possessing a general axis (A-A') making an angle ($\alpha$) with the axis (B-B') of the neck, the prosthesis being **characterized in that**

   · in projection onto a frontal plane F, the angle $\alpha$ possesses a value, expressed in degrees, that satisfies the following relationship:

$$\alpha = 0.1376\ L + 108.3648 \pm e$$

   where:

   · L is the length of the stem (1) measured parallel to the axis A-A' between a shoulder (18) perpendicular to the axis A-A' and the bottom end of the stem (1);
   · e is a tolerance of value 0.3°, preferably 0.16°, and in more particularly preferred manner 0.09°;

   . the neck (2) possesses a length lc, expressed in millimeters and measured between its end remote from the stem (1) and the base connecting the neck to the stem, that satisfies the following relationship:

$$lc = 0.248\ L - 1.5 \pm e'$$

   where:

   · e' is a tolerance of value 0.6 mm, preferably 0.3 mm, and in more particularly preferred manner 0.25 mm.

2. A hip prosthesis according to claim 1, **characterized in that** the value of the angle $\alpha$ lies in the range 124° to 132° and, preferably, in the range 125.3° to 130.6°.

3. A hip prosthesis according to claim 1 or claim 2, **characterized in that** the length lc of the neck (2) presents a value in the range 31 mm to 43 mm, and preferably in the range 32 mm to 42 mm.

4. A hip prosthesis according to any one of claims 1 to 3, **characterized in that** it has a "lateral" edge (17) starting at a shoulder (18) that is substantially perpendicular to the axis A-A' of the neck, the edge extending to the bottom end of the tail (6) and being of generally convex shape.

5. A hip prosthesis according to claim 4, **characterized in that** the lateral edge (17) has a proximal portion (19) and a distal portion (20) that, when seen in profile, form an obtuse angle $\beta$ that in projection onto a frontal plane F presents a value in the range 161° to 180° and, preferably, in the range 162.1° to 178.9°.

6. A hip prosthesis according to claim 5, **characterized in that** the value, expressed in degrees, of the angle $\beta$ satisfies the following relationship:

$$\beta = 9.1584\ L + 166.8509 \pm e''$$

where:

· L is the length of the stem (1) measured parallel to the axis A-A' between a shoulder (18) perpendicular to the axis A-A' and the bottom end of the stem (1);
· e" is a tolerance of value 6.6°, preferably 5.5°, and in more particularly preferred manner 3.9°.

7. A hip prosthesis according to claim 5 or claim 6, **characterized in that** the width $\ell$ of the stem (1), measured perpendicularly to the axis A-A' from the junction (27) between the proximal and distal portions (19, 20) of the lateral edge (17) and as far as an opposite medial edge (11), presents a value in the range 16 mm to 31 mm and, preferably, in the range 17 mm to 29.4 mm.

8. A hip prosthesis according to claim 7, **characterized in that** the width t of the stem (1), expressed in millimeters, satisfies the following relationship:

$$\ell = 0.323\ L - 22.852 \pm e'''$$

where:

· L is the length of the stem (1) measured parallel to the axis A-A' between a shoulder (18) perpendicular to the axis A-A' and the bottom end of the stem;
· e''' is a tolerance of value 0.6 mm, preferably 0.4 mm, and in more particularly preferred manner 0.25 mm.

9. A hip prosthesis according to any one of claims 1 to 8, **characterized in that** the neck has two flats (25, 26), respectively an anterior flat and a posterior flat, that extend from a plane connection base between the neck (2) and the stem (1) towards an opposite end of the neck (2).

10. A hip prosthesis according to any one of claims 1 to 9, **characterized in that** the length L of the stem (1) lies in the range 125 mm to 162 mm.

11. A hip prosthesis according to any one of claims 1 to 10, **characterized in that** the end of the neck (2) forms a cone for fitting the joint head.

12. A set of hip prostheses of different sizes according to any one of claims 1 to 11, the set comprising at least two hip prostheses of different stem lengths, the length L of the stem of each of the prostheses of the set belonging to a different category selected from the following categories:

EP 1 958 596 B1

· T1: stem length L in the range 125 mm to 126 mm;
· T2 stem length L in the range 128 mm to 129 mm;
· T3: stem length L in the range 131 mm to 132 mm;
· T4 stem length L in the range 136 mm to 137 mm;
· T5 stem length L in the range 141 mm to 142 mm;
· T6: stem length L in the range 147 mm to 148 mm;
· T7: stem length L in the range 152 mm to 153 mm;
· T8: stem length L in the range 156 mm to 158 mm;
· T9: stem length L in the range 161 mm to 162 mm;

the length L of the stem (1) being measured parallel to the axis A-A' between a shoulder of the neck perpendicular to the axis A-A' and the bottom end of the stem.

**FIG.1**

FIG.2